# EUROPEAN PATENT APPLICATION

(11) **EP 2 233 072 A1**
(43) Date of publication of application: **29.09.2010**
(21) Application number: 09156392.4
(22) Date of filing: 27.03.2009
(51) Int. Cl.: A61B 5/103

(54) **Method and device for monitoring a motion sequence of a person**

(71) Applicant: Koninklijke Philips Electronics N.V., 5621 BA Eindhoven (NL); Philips Intellectual Property & Standards GmbH, 20099 Hamburg (DE)
(72) Inventor: The designation of the inventor has not yet been filed
(74) Representative: Golla-Franz, Anke Lucia

(57) **Abstract**

The present invention relates to a method and a device for monitoring a motion sequence of a person by comparing a first motion pattern (2) and a second motion pattern (4), wherein the first and second motion pattern are provided by a sensor and wherein the first motion pattern preferably relates to a first footstep and the second motion pattern preferably relates to a second footstep.

## Description

The present invention relates to a method and a device for monitoring a motion sequence of a person by comparing a first motion pattern and a second motion pattern, wherein the first and second motion pattern are provided by a sensor and wherein the first motion pattern preferably relates to a first footstep and the second motion pattern preferably relates to a second footstep.

It has become common knowledge that the body motion of a human subject can be monitored by the aid of inertial sensors. For instance, the prior art document US2007/0270214 Al discloses a method for analyzing and improving the performance of a body motion of a human subject with inertial sensors by monitoring a body motion of interest, converting the sensor data into motion data and animation, comparing the motion data with existing data for motion related performance parameters, providing a data display of the results and based on the results prescribing a training regime with exercises selected from a library of standardized exercises. It is highly disadvantageous that this method requires the comparison of the motion data with existing data, because the motion pattern of every single body motion is very complex and strongly deviates with different persons. Therefore, it is not possible to generate suchlike existing data to detect the quality or the efficiency of motion sequences of different persons, in general.

Starting from this prior art, it is the object of the present invention to provide a method for monitoring a motion sequence of a person that does not have the drawbacks mentioned in connection with the prior art.

The above object is accomplished by a method for monitoring a motion sequence comprising the steps of determining a measuring signal with a sensor, determining a first motion pattern in the measuring signal, determining a second motion pattern in the measuring signal, comparing the first and the second motion pattern and providing a motion signal in dependance of said comparison.

Beneficially, the motion signal is provided without the aid of existing data or the like, because the method according to the present invention generates the comparative data itself. The measuring signal is divided into a first motion pattern relating to first body motion and into a second motion pattern relating to second body motion. A motion pattern in the sense of the present invention refers to a repetitive pattern in the measuring signal which depends on a repetitive pattern of movement of the user or of a body part of the user. The motion signal depends on the comparison between the first and the second motion pattern and therefore relates to an asymmetry between the first and the second body motion. As many main body motions, like running, walking, swimming (in particular crawling) or the like, can be divided into two symmetrical body motions, one for the left leg and the other one for the right leg for instance, the symmetry of these two body motions are an indicator for the quality, the efficiency and/or the healthiness of the main body motion. For example, the performance of a competitive athlete could be analyzed and if necessary highly increased by monitoring the motion sequence. Alternatively, the strength and the flavor of a limping of a limping person depend on the symmetry of the motion patterns between a left footstep and a right footstep, for example. In this case, the method according to the present invention allows to quantitatively analysis the walking pattern of the person and give him and in particular his therapist valid diagnostic and "in a closed loop feedback scheme" therapeutic information about the limping strength and flavor. For example, during a person's walk, the sensor transmits his orientation and/or position in space to a processing unit. The characteristic period for a walking movement consists of two single footsteps, one extending the left leg and one extending the right leg. For a normal healthy subject the correlation of subsequent double footsteps and the correlation of subsequent single footsteps are comparatively high, whereas for a limping person the correlation of subsequent double footsteps are also comparatively high, but the correlation of subsequent single footsteps are significantly lower. The lower correlation between the subsequent single footsteps is detected by comparing the first and the second motion pattern. Beneficially, the correlation is quantified by the motion signal, wherein the dysfunction of the musculoskeletal system, the limping of the person and/or the course of a medical therapy could be derived from the motion signal.

It is to be understood that the present invention does not only refer to walking patterns, but as mentioned above also to every symmetric body motion of the person. The wording "determining" the measuring signal means measuring the measuring signal and/or processing a measured signal to the measuring signal. It is conceivable, that not the measured signal itself is used to find the motion patterns, but a measured signal which is generated by some mathematical operation, e.g. applying geometric correction turns to the original measured signal, or calculating the derivative of the measured signal. E.g. in the case of magnetometers, there might be a 2*pi ambiguity that has to be resolved first.

The sensor shall be able to measure its orientation, its position in space and/or acceleration. In a preferred embodiment the sensor comprises an inertia sensor, a magnetometer, an acceleration sensor, a gyroscope, a positioning sensor, an altimeter or the like. In particular, the inertial sensor is attached to the person in his sagittal plane, as a strap around his chest (like a polar belt, with the sensor in the middle), on his back on the spine or on the top of his head (e.g. in a cap or a headband), for example. Beneficially, the orientation of the sensor is unimportant for the analysis of the walking pattern, as long as the sensor is placed in the sagittal plane. The sensor shall be able to measure its position, its orientation, its velocity and/or its acceleration in space. A favorable embodiment uses a wireless data transmission from the sensor to a sender-receiver unit connected to a data processing device (e.g. Bluetooth, WLAN or the like), but arrangements with other means of transmission are also suitable (e.g. infrared, cable, ultrasound). The data processing device particularly comprises a cellular phone, a personal digital assistant (PDA) and/or a personal computer of the person and/or of his therapist. In a preferred embodiment the first motion pattern, the second motion pattern, the measuring signal and/or the motion signal is transmitted directly to a service center of the therapist via a cellular phone network, for instance. Furthermore, the measuring signal, the first motion pattern, the second motion pattern, a time lag, a correction term, the motion signal and/or personal data about the person are stored in a memory unit and/or entered manually to the processing unit with the aid of an input unit.

In a preferred embodiment the first motion pattern comprises a first interval about a first maximum in the measuring signal and the second motion pattern comprises a second interval about a second maximum in the measuring signal. Beneficially, the first and the second maximum, the first and the second interval about the first and the second maximum are allocated to a first and a second body motion, like a first and a second footstep (left footstep and right footstep, first left footstep and second left footstep or first right footstep and second right footstep) and/or like a first and a double footstep (first left and right footstep and second left and right footstep) for instance. The first and the second motion pattern are applicable for comparing the first and the second body motion in order to quantify the symmetry between the first and the second body motion. The motion signal therefore indicates the strength and the flavor of a limping of the person. Preferably, an interval about a first and a subsequent second maximum in the measuring signal is identified as the first motion pattern and an interval about a third and a subsequent fourth maximum in the measuring signal is identified as the second motion pattern, wherein the first and the second maximum comprises a first double footstep and the third and the fourth maximum comprises a second double footstep.

In a preferred embodiment further first motion patterns und further second motion patterns in the measuring signal are determined, wherein the motion signal is provided in dependance of a comparison of multiple further first motion patterns and multiple further second motion patterns, and/or an average first motion pattern is determined from multiple further first motion patterns and an average second motion pattern is determined from multiple further second motion patterns. Beneficially, the averaging of the motion patterns and/or of the motion signal leads to an increased accuracy of the motion signal.

In a preferred embodiment a time lag between the first motion pattern and the second motion pattern and/or between a first motion pattern and a further first motion pattern is determined, wherein further first and/or further second motion patterns are determined in consideration of the time lag. Beneficially, the determination of the above mentioned time lag leads to an easier and more efficient determination of the second motion pattern and/or of further first and second motion pattern.

Particularly, the orientation of the sensor relatively to the sagittal plane is optimized by calibration measurements to increase the accuracy of the method according to the present invention. For instance, the orientation of the sensor is optimized visually by the therapist. Alternatively, the orientation of the sensor relatively to the sagittal plane could be verified by a movement of the person parallel to the sagittal plane. For this purpose, the person sits on a chair and bends himself sequentially forward and rearwards. The optimal position of the sensor is adjusted in dependence of the derivation in the measuring signal. Alternatively, a correction term is determined in dependance of a derivation of the orientation of the sensor relatively to the sagittal plane. In this case, the motion signal is provided in dependance of said correction term.

Preferably, the dysfunction of the musculoskeletal system, a limping of the person and/or the course of a medical therapy is derived from the motion signal.

Another object of the present invention is a device for monitoring a motion sequence of a person comprising:
- a sensor for determining a measuring signal
- a processing unit for determining a first motion pattern and a second motion pattern in the measuring signal,
- a comparator for comparing the first and the second motion pattern and
- an output unit for displaying a motion signal provided by the comparator.

Advantageously, the device for monitoring a motion sequence of a person give the user and/or a therapist of the user directly and in particular just in time a feedback of the course of the user's movement. In particular, the motion signal is determined by an above mentioned method for monitoring a motion sequence according to the present invention. Preferably, the output unit comprises a display for displaying the feedback depending on the motion signal to the user and/or to a therapist. The output unit can be wireless connected and/or telephone linked to the comparator. In a preferred embodiment the device comprises a memory unit for storing the first motion pattern, the second motion pattern, the measuring signal and/or the motion signal. Particularly, the sensor is provided in the sagittal plane of the person and particularly in the median plane of the person. The sensor preferably comprises an inertia sensor, a gyroscope, a magnetometer, an accelerometer, a positioning sensor, an altimeter and/or the like.

These and other characteristics, features and advantages of the present invention will become apparent from the following detailed description, taken in conjunction with the accompanying drawing, which illustrates, by way of example, the principles of the invention. The description is given for the sake of example only, without limiting the scope of the invention. The reference figures quoted below refer to the attached drawing.
- Figures 1a and 1b: schematically illustrate a measured measuring signal and a corrected measuring signal for a symmetric gait of a person in accordance with the present invention
- Figures 2a and 2b: schematically illustrates a measured measuring signal and a corrected measuring signal for a limping gait of a person in accordance with the present invention

The present invention will be described with respect to particular embodiments and with reference to a certain drawing but the invention is not limited thereto but only by the claims. The drawing described is only schematic and is non-limiting. In the drawing, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

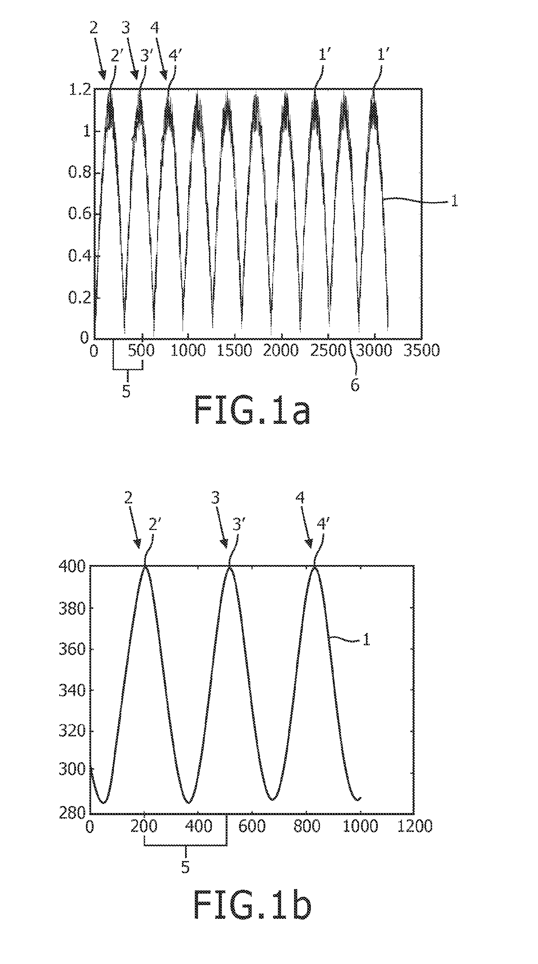

In Figures 1a a measured measuring signal 1 of a method according to the present invention in dependance of time 6 is shown, wherein the measuring signal 1 is measured by an inertia sensor, which is provided in the sagittal plane of a person. The peaks 1' of the measuring signal 1 relates to different body movements of the person, particularly either to a left or a right single footstep of the person. In a first step of the above mentioned method orientation deviations of the sensor relatively to the sagittal plane are removed by calibration steps, wherein the orientation of the sensor is optimized visually by the therapist of the person and/or during a movement of the person parallel to the sagittal plane. In a second step the orientation of the sensor is made invariable to mirroring on the sagittal plane, e.g. by mirroring all orientations of the sensor that cross the sagittal plane with respect to just this plane.

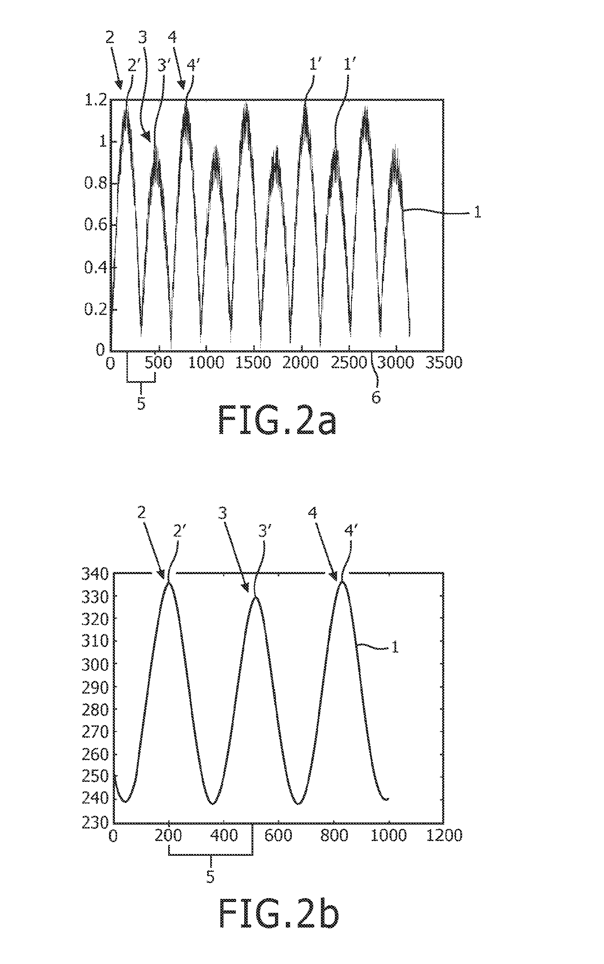

In Figure 2a a corrected measuring signal 1 based on the measuring signal illustrated in figure 1a is shown. In a third step of the method according to the present invention a peak detection algorithm determines a time leg 5 based on the maximum deviation of the sensor position difference to the starting position. The time leg 5 corresponds to a footstep rate of the person, which typically accounts between one and two seconds. Furthermore, the corrected measuring signal 1 is filtered by a low band-pass filter. Based on the time leg 5 a first and a second maximum 2', 3' of the measuring signal 1 are isolated in a fourth step of the method according to the present invention, wherein an interval about the first maximum 2' is determined as a first motion pattern 2 and an interval about the second maximum 3' is determined as a second motion pattern 3. The first and the second maximum 2', 3' and accordingly the first and the second motion pattern 2, 3 in the corrected measuring signal 1 are shown in figure 2a. The first motion pattern 2 can be allocated to a first single footstep and the second motion pattern 3 to a second single footstep. The third maximum 4' in the corrected measuring signal 1 is determined as a further first motion pattern 4 and accordingly corresponds to a further first single footstep. In a fifth step of the method according to the present invention the first and the second motion patterns 2, 3 are compared to each other to derive the correlation between the first and the second single footstep. A motion signal which is provided in dependance of the differences between the first and the second motion pattern 2, 3, in particular between the height of the first and the second maximum 2', 3', is therefore a measure for a limping of the person. Alternatively, the correlation between a first double footstep and a second double footstep of the person is derived from the comparison of the first and the further first motion pattern 2, 4. The course of the measuring signals 1 of figure 2a shows that the person has an essentially symmetrical gait without any limping deviations, because the height of the different maxima for the left and the right single footsteps is almost constant.

In Figures 1b and 2b a measured measuring signal 1 and a corrected measuring signal 1 of a limping person are shown, wherein the first, second, third, fourth and fifth steps of the method according to the present invention are accomplished similar to the measuring signal 1 illustrated in figures 1a and 2a. In contrast to figures 1a and 2c, the illustrated measuring signals 1 comprise a difference between the height of the first maximum 2' and the height of the second maximum 3'. This difference relates to an asymmetry between the first and the second single footsteps of the person and is therefore a measure for a limping of the person. However, the correlation between the first and the further first motion pattern 2, 4 are similar to a correlation between the first and the third motion pattern 2, 4 of a non-limping person as shown in figure 2a.

## Claims

1. Method for monitoring a motion sequence of a person comprising the steps of
- determining a measuring signal (1) with a sensor,
- determining a first motion pattern (2) in the measuring signal (1),
- determining a second motion pattern (3) in the measuring signal (1),
- comparing the first and the second motion pattern (2, 3) and
- providing a motion signal in dependance of said comparison.

2. Method according to claim 1, **characterized in that** an interval about a first maximum (2') in the measuring signal (1) is identified as the first motion pattern (2) and an interval about a second maximum (3') in the measuring signal (1) is identified as the second motion pattern (3).

3. Method according to claim 1, **characterized in that** an interval about a first and a subsequent second maximum in the measuring signal (1) is identified as the first motion pattern (2) and an interval about a third and a subsequent fourth maximum in the measuring signal is identified as the second motion pattern (3).

4. Method according to one of the preceding claims, **characterized in that** further first motion patterns (4) und further second motion patterns in the measuring signal (1) are determined, wherein the motion signal is provided in dependance of a comparison of multiple further first motion patterns and multiple further second motion patterns.

5. Method according to claim 4, **characterized in that** an average first motion pattern is determined from multiple further first motion patterns and an average second motion pattern is determined from multiple further second motion patterns.

6. Method according to one of the preceding claims, **characterized in that** a time lag (5) between the first motion pattern (2) and the second motion pattern (3) is determined.

7. Method according to claim 6, **characterized in that** further first and/or further second motion patterns (4) are determined in consideration of the time lag (5).

8. Method according to one of the preceding claims, **characterized in that** first and further first motion patterns (2, 4) are allocated to first footsteps of the person and second and further second motion patterns (3) are allocated to second footsteps of the person.

9. Method according to one of the claims 1 to 7, **characterized in that** the first motion patterns (2) are allocated to first double footsteps of the person and the second motion patterns (3) are allocated to second double footsteps of the person.

10. Method according to one of the preceding claims **characterized in that** a correlation between the first and the second footsteps and/or between the first and the second double footsteps is determined.

11. Method according one of the preceding claims, **characterized in that** the orientation of the sensor relatively to the sagittal plane is optimized by calibration measurements.

12. Method according to one of the preceding claims, **characterized in that** a correction term is determined in dependance of a derivation of the orientation of the sensor relatively to the sagittal plane, wherein the motion signal is provided in dependance of said correction term.

13. Method according to one of the preceding claims, **characterized in that** the measuring signal (1), the first motion pattern (2), the second motion pattern (3), the time lag (5), the correction term, the motion signal and/or personal data about the person are stored in a memory unit and/or entered manually with the aid of an input unit.

14. Device for monitoring a motion sequence of a person comprising:
- a sensor for determining a measuring signal
- a processing unit for determining a first motion pattern and a second motion pattern in the measuring signal,
- a comparator for comparing the first and the second motion pattern and
- an output unit for displaying a motion signal provided by the comparator.

15. Device according to claim 14, **characterized in that** the output unit comprises a display for displaying a feedback depending on the motion signal to the user and/or to a therapist.
